(19) Europäisches Patentamt
European Patent Office
Office européen des brevets

(11) EP 1 941 864 A1

(12) DEMANDE DE BREVET EUROPEEN

(43) Date de publication:
**09.07.2008 Bulletin 2008/28**

(21) Numéro de dépôt: **08150029.0**

(22) Date de dépôt: **03.01.2008**

(51) Int Cl.:
***A61K 8/81*** *(2006.01)* ***A61K 8/73*** *(2006.01)*
***A61K 8/891*** *(2006.01)* ***A61K 8/19*** *(2006.01)*
***A61Q 1/10*** *(2006.01)*

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(30) Priorité: **04.01.2007 FR 0752524**

(71) Demandeur: **L'ORÉAL**
**75008 Paris (FR)**

(72) Inventeurs:
- **Auguste, Frédéric**
**94550, CHEVILLY-LARUE (FR)**
- **Fouron, Jean-Yves**
**92340, BOURG-LA-REINE (FR)**

(74) Mandataire: **Le Coupanec, Pascale A.M.P.**
**Nony & Associés,**
**3 rue de Penthièvre**
**75008 Paris (FR)**

(54) **Kit de maquillage des fibres kératiniques**

(57) La présente invention concerne un kit de maquillage des fibres kératiniques comprenant au moins :
- une première composition présentant une énergie de surface inférieure ou égale à 30 mN/m, et
- une deuxième composition présentant une énergie de surface, telle que la différence entre cette énergie de surface et celle de la première composition est supérieure ou égale à 5 mN/m, et une viscosité comprise entre 0,001 et 2 Pa.s.

Fig.3

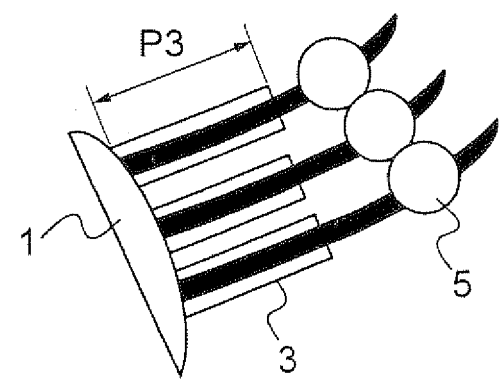


EP 1 941 864 A1

## Description

La présente invention concerne un kit de maquillage des fibres kératiniques et le procédé de maquillage correspondant.

La présente invention se rapporte au maquillage des fibres kératiniques, comme les cils, les sourcils et les cheveux, et plus particulièrement au maquillage des cils.

Le kit de maquillage selon l'invention peut se présenter sous la forme d'un kit de maquillage pour les cils, pour les sourcils, ou d'un kit de maquillage des cheveux. Plus spécialement, l'invention porte sur un mascara. Il peut notamment s'agir d'un kit de maquillage des cils.

Il existe en pratique essentiellement deux types de formulation de mascara, à savoir d'une part, des mascaras à phase continue aqueuse, dits « mascaras émulsion », se présentant sous forme d'émulsion de cires dans l'eau et, d'autre part, des mascaras à phase continue solvant ou huile, anhydres ou à faible teneur en eau et/ou solvants hydrosolubles, dits « mascaras waterproof », formulés à l'état de dispersion de cires dans des solvants non aqueux. Par ailleurs, certains mascaras se présentent sous forme d'émulsions, de cire dans l'eau, qui sont également qualifiés de « waterproof ». Ces dernières compositions se caractérisent par la présence d'au moins un latex ou pseudo-latex, à savoir d'une suspension colloïdale d'un polymère filmogène, qui confère la résistance à l'eau.

Classiquement, les mascaras comprennent au moins un polymère filmogène permettant notamment un gainage des cils.

Le but recherché par la présente invention est de disposer d'un dispositif unique permettant d'offrir aux utilisateurs de mascara le moyen de réaliser des effets de maquillage variés, facilement modulables suivant la manière dont l'application est opérée.

Il est connu du document EP 953 332 une composition de maquillage comprenant au moins une dispersion aqueuse de particules de polymère filmogène et au moins un agent épaississant, caractérisé par le fait que ladite composition présente une viscosité, mesurée à un cisaillement de 500 $s^{-1}$, allant de 0,001 Pa.s à 2 Pa.s., ainsi qu'un procédé de maquillage des fibres kératiniques associé permettant la formation de perles réparties le long des fibres kératiniques, suite à une application selon une vitesse adaptée.

Il est par ailleurs connu du document FR 2 844 706 des compositions permettant d'obtenir, après application sur les fibres kératiniques, des gouttes, de préférence transparentes, caractérisées par la présence d'un polymère particulier.

Les inventeurs ont constaté qu'il est possible d'obtenir une large gamme de types de maquillage des fibres kératiniques par application successive de deux compositions définies respectivement, pour la première d'entre elles, par une énergie de surface maximale de 30 mN/m et, pour la deuxième d'entre elles, par une énergie de surface présentant une différence de valeur d'énergie de surface supérieure ou égale à 5 mN/m par rapport à l'énergie de surface de la première composition, et une viscosité comprise entre 0,001 et 2 Pa.s.

Des exemples de rendu de maquillage sont schématisés dans les figures annexées.

En d'autres termes, la mise en oeuvre du kit de maquillage selon la présente invention permet de moduler tant en quantité qu'en localisation sur les fibres kératiniques le dépôt de matière souhaité le long desdites fibres kératiniques. En effet, les caractéristiques des première et deuxième compositions engendrent le déplacement de matière par capillarité, qui, après séchage, donc durcissement, figent le rendu maquillage sous forme par exemple de gouttelettes uniques ou multiples sur chaque cil pris séparément, ou encore de « ponts » entre les fibres kératiniques. Il y a ainsi formation d'un « excès de matière » qui peut présenter ou non le même effet optique et notamment la même couleur que celui de la première composition, qui procure un résultat maquillage particulier comparativement à celui obtenu avec les mascaras courants, qui au contraire visent généralement un maquillage homogène le long de chaque fibre kératinique.

Le procédé de maquillage selon la présente invention peut aussi être utilisé pour le maquillage des cils de sorte à souligner le regard grâce à une impression de volume déposé important. En particulier, le dépôt important de matière situé à un endroit précis le long des cils, obtenu grâce à la mise en oeuvre du procédé selon la présente invention, permet de parvenir à cette impression.

Ainsi, selon un premier aspect, la présente invention concerne un kit de maquillage des fibres kératiniques comprenant au moins :

- une première composition présentant une énergie de surface inférieure ou égale à 30 mN/m,
- une deuxième composition présentant une énergie de surface de la composition avant séchage, telle que la différence entre cette énergie de surface et celle de la première composition est supérieure ou égale à 5 mN/m, et une viscosité comprise entre 0,001 et 2 Pa.s.

Selon un deuxième aspect, la présente invention concerne un procédé de maquillage et/ou de soin non thérapeutique des fibres kératiniques, caractérisé en ce qu'il comprend au moins une étape d'application d'une première composition conforme à la présente invention sur tout ou partie des fibres kératiniques, formant un premier dépôt, et

au moins une étape d'application d'une deuxième composition conforme à la présente invention sur tout ou partie du premier dépôt et/ou des fibres kératiniques.

Dans le cadre de la présente invention, par « fibres kératiniques » on entend notamment les cheveux, les cils et les sourcils.

Les kits de maquillage conformes à l'invention comprennent un milieu physiologiquement acceptable, notamment cosmétiquement acceptable, c'est-à-dire un milieu compatible en particulier avec les fibres kératiniques telles que les cheveux, les cils, les sourcils.

Par « cosmétiquement acceptable », on entend, dans le cadre de la présente invention, un composé dont l'utilisation est compatible avec une application sur les matières kératiniques.

La manifestation du rendu maquillage recherché dans le cadre de la présente invention s'observe notamment lorsque la deuxième composition est déposée successivement à la première composition. Ainsi, il faut au moins deux gestes de l'application pour obtenir l'effet maquillage recherché.

L'application des deux compositions peut être effectuée à l'aide de tout moyen d'application, ou applicateur, tel que cela est décrit ci-après.

La première et la deuxième compositions peuvent également être respectivement qualifiées de « basecoat » et de « topcoat ».

Par « dépôt sec » on entend dans la suite de la description le dépôt formé après application et séchage d'une composition sur un support, notamment après séchage de ce dépôt pendant 24 heures à température ambiante (23 +/- 2 °C), et humidité relative contrôlée HR 50 +/- 5 %.

Plus particulièrement, la présente invention concerne un kit de maquillage des fibres kératiniques comprenant au moins :

- une première composition présentant une énergie de surface mesurée sur dépôt sec, inférieure ou égale à 30 mN/m, et
- une deuxième composition présentant une énergie de surface mesurée avant séchage, telle que la différence entre cette énergie de surface et celle de la première composition mesurée sur dépôt sec, est supérieure ou égale à 5 mN/m, et une viscosité comprise entre 0,001 et 2 Pa.S.

**Mesure de l'énergie de surface**

L'énergie de surface peut être mesurée de différentes manières.

Pour la première composition, l'énergie de surface est par exemple mesurée sur dépôt sec. La mesure est alors effectuée de la façon suivante :

On réalise des dépôts de la première composition d'épaisseur 100 μm humide sur le porte échantillon en bakélite d'un tensiomètre à angle de contact tel que le tensiomètre DAT 1100, commercialisé par la Société Fibro (Suède).

Le dépôt de la première composition est réalisé en posant à plat sur le porte échantillon du tensiomètre un pochoir métallique rectangulaire de dimensions 10 X 20 mm et d'épaisseur 100 μm ; On remplit l'intérieur du cadre du pochoir avec la composition et on arase la surface à l'aide d'une lame de microscope standard (lame en verre, dimensions 26 X 76 mm), afin d'enlever l'excédent de matière.

Après séchage de ces dépôts pendant 24 heures à température ambiante (23 +/-2 °C), et humidité relative contrôlée HR 50 +/- 5 %, les mesures d'angles de contact sont réalisées à 20 +/- 1 °C et 50 % d'humidité relative avec l'eau avec au moins 3 des solvants suivants : eau, glycérol, huile de parleam, diiodométhane et formamide.

Pour chacun des solvants, une goutte de solvant, de volume compris entre 2 et 10 microlitres, est déposée sur le dépôt sec formé par la première composition. Les systèmes optiques et d'analyse d'images du tensiomètre permettent de suivre l'évolution au cours du temps de l'étalement de la goutte. L'angle de contact du liquide sur le dépôt sec formé par la première composition correspond à l'angle compris entre la surface de contact composition/liquide et la tangente à la goutte passant par le point de rencontre de la goutte, de l'air et de la surface. 2 angles sont mesurés, un de chaque côté de la goutte, l'ordinateur calcule la moyenne de ces 2 angles.

La valeur de l'angle de contact utilisée pour la détermination de l'énergie de surface critique est la moyenne des angles de contact mesurés pour 5 à 10 gouttes sur le dépôt formé par la première composition, les valeurs de l'angle étant prises après un temps de contact entre la goutte de solvant et le dépôt formé par la première composition égal à 1 seconde.

Pour le calcul de l'energie de surface de chaque première composition, on utilise le modèle d'Young avec les équations de Fowkes (cf « Determination of interfacial tensions, contact angles, and dispersion forces in surfaces by assuming additivity of intermolecular interactions in surfaces » Fowkes, F. M. Journal of Physical Chemistry (1962), 66, 382 et « Contact angle, wettability & adhesion, advances in chemistry series », N°43 Fowkes (1964)).

Connaissant les composantes dispersives et polaires des solvants (respectivement $\gamma_L^d$ et $\gamma_L^p$ exprimés en

$mJ/m^2$ avec également l'énergie superficielle γL définie par $\gamma_L = \gamma_L^d + \gamma_L^p$) et l'angle de contact θ mesuré entre le solvant et le dépôt de composition, on trace un graphique $\frac{\gamma L(1+\cos\theta)}{2\sqrt{\gamma_L^d}}$ en fonction de $\sqrt{(\gamma_L^p/\gamma_L^d)}$.

L'allure de ce graphique s'approche d'une droite de pente légèrement positive ; la pente de cette droite correspond à $\sqrt{(\gamma_S^p)}$ et l'ordonnée à l'origine à $\sqrt{(\gamma_S^d)}$. On obtient ainsi les valeurs de $\gamma_S^d$ et $\gamma_S^p$ c'est à dire les composantes dispersive et polaire de l'énergie libre de surface du dépôt formé par la première composition.

L'énergie de surface de la composition $\gamma_S$ est défini par la relation : $\gamma_S = \gamma_S^d + \gamma_S^p$.

Pour la seconde composition, l'énergie de surface est mesurée avant séchage de la composition, autrement dit sur la formule à l'état liquide, selon la méthode de la lame de Wilhelmy décrite dans la norme ISO 304-1985 (F), en utilisant un corps de mesure en platine tel que cité dans la partie 5.2 a). La mesure est réalisée avec le tensiomètre K12 de Krüss à une température de 20 °C, la profondeur d'immersion de la lame dans la composition est fixée à 2 mm, les autres paramètres sont choisis par le logiciel de l'appareil afin d'éviter la rupture du film interfacial.

Dans le cas des liquides, l'énergie de surface est aussi appelée tension de surface. Le nettoyage de la lame et de la verrerie pour le tensiomètre est détaillé ci-après pour ce protocole.

Après nettoyage avec un solvant approprié (acétone, par exemple), la lame est passée à la flamme d'un bec Bunsen jusqu'à ce qu'elle rougisse. Pour ce qui est de la verrerie (cristallisoir), elle est immergée dans une solution de détergent de type DECON pendant 12 heures. Elle est ensuite rincée plusieurs fois avec de l'eau ultrapure et séchée au décapeur thermique.

La qualité du nettoyage est vérifiée par une mesure de la tension de surface d'eau ultrapure. On doit trouver 72.3 +/- 0.5 mN/n.

## Mesure de la viscosité

La viscosité est mesurée à 25°C ± 0,5 °C à l'aide d'un rhéomètre à contrainte imposée Haake RS600 de la société Thermo Rhéo équipé d'un mobile de géométrie cône/plan d'un diamètre compris entre 2 cm et 6 cm et d'un angle compris entre 1° et 2°, le choix du mobile étant fonction de la viscosité à mesurer (plus la formule est fluide, plus le diamètre du cône choisi est grand et plus l'angle est petit). La mesure est effectuée en imposant à l'échantillon d'huile une rampe logarithmique de gradient de cisaillement $\dot{\gamma}$ allant de $10^{-3}s^{-1}$ à 1000 $s^{-1}$, pendant une durée de 5 minutes. Puis on trace le rhéogramme représentant l'évolution de la viscosité en fonction du gradient de cisaillement $\dot{\gamma}$. La valeur retenue est la valeur de la viscosité à 500 $s^{-1}$, qu'elle soit mesurée à ce gradient ou extrapolée par le tracé si aucun point expérimental ne correspond à cette valeur.

## PREMIERE COMPOSITION

La première composition présente une énergie de surface inférieure ou égale à 30 mN/m.

Selon un mode de réalisation préféré, l'énergie de surface de la première composition est inférieure ou égale à 25 mN/m, et de façon encore plus préférée inférieure à 20 mN/m.

Afin d'atteindre une telle énergie de surface, la première composition comprend avantageusement des composés siliconés et/ou fluorés, notamment choisis parmi les cires siliconées et/ou fluorées, les pigments présentant un enrobage ou un traitement de surface siliconé et/ou fluoré et leurs mélanges.

La composition peut notamment comprendre des cires fluorées et/ou siliconées ou encore des cires comprenant des parties siliconées et/ou fluorées.

Concernant la définition générale des cires on se reportera au paragraphe qui leur est dédié dans la description ci-après.

### Cires fluorées

On entend par cire fluorée un composé comportant un squelette carboné et un nombre d'atomes de fluor de préférence supérieur à celui des atomes d'hydrogène, le squelette carboné pouvant être interrompu par au moins un atome d'oxygène. Cette cire ne comporte que des atomes de fluor, d'hydrogène et de carbone et éventuellement d'oxygène.

Parmi les cires fluorées à squelette carboné, on peut citer notamment les esters fluorés répondant à la formule (VI) suivante :

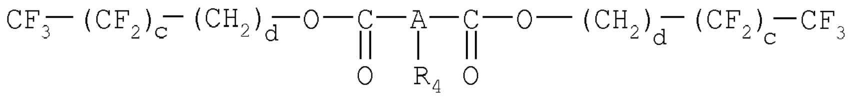 (VI)

dans laquelle :

$R_4$ représente un atome d'hydrogène ou le radical

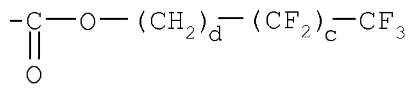 ,

A représente une chaîne hydrocarbonée, alkylène ou alcénylène,ladite chaîne étant linéaire ou ramifiée, éventuellement hydroxylée, et comprenant de 1 à 18 atomes de carbone,
c va 1 à 17, et
d va 1 à 18.

Parmi les composés de formule (VI), on peut notamment citer le dodécane 1,12,dioate de 2F-octyl-éthyle de formule suivante :

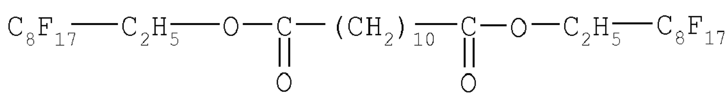

et les tri-citrates de perfluoroalkyle en $C_1$-$C_6$, plus particulièrement le tri-citrate de perfluorobutyle vendu sous la dénomination de "ZONYL TBC®" par la Société DUPONT. Comme autres cires fluorées utilisables dans l'invention, on peut citer les cires de polytétrafluoroéthylène (PTFE).

De préférence, on utilise des cires ayant un point de fusion allant de 45 °C à 150 °C, notamment comme les esters fluorés de formule (VI).

On peut également utiliser des microdispersions de cires fluorées comme par exemple la « Microdispersion 411 » de MicroPowders, qui est un mélange de cires de polyéthylène, et le produit « Aquapolyfluo 411 » de MicroPowders, qui est un mélange de cire de polyéthylène et de polytétrafluoroéthylène.

Cires siliconées

Les cires siliconées utilisables dans la composition selon l'invention sont avantageusement des polysiloxanes substitués, de préférence à bas point de fusion. Il s'agit notamment de polysiloxanes linéaires substitués constitués essentiellement (les groupes terminaux mis à part) de motifs de formules (II) et (III), dans les proportions molaires respectives m et n :

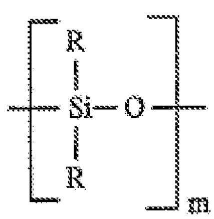 et 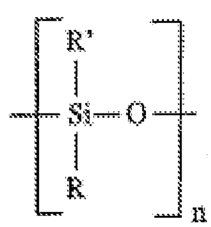

(II) (III)

dans lesquelles :

- chaque substituant R est défini comme précédemment,
- chaque R' représente indépendamment une chaîne hydrocarbonée linéaire ou ramifiée, saturée ou insaturée, comprenant de 6 à 30 atomes de carbone, ou bien un groupement -X-R", chaque X représentant indépendamment :

  -O-,

  $-(CH_2)_a-O-CO-$,

  $-(CH_2)_b-CO-O-$,

  a et b représentent indépendamment des nombres pouvant varier de 0 à 6, et chaque R" représente indépendamment une chaîne hydrocarbonée, saturée ou insaturée, comprenant de 6 à 30 atomes de carbone,
- m est un nombre pouvant varier de 0 à 400, et en particulier de 0 à 100,
- n est un nombre pouvant varier de 1 à 200, et en particulier de 1 à 100,

la somme (m + n) étant inférieure à 400, et en particulier inférieure ou égale à 100.

Ces cires de silicones sont connues ou peuvent être préparées selon les méthodes connues. Parmi les cires de silicones commerciales de ce type, on peut citer notament celles vendues sous les dénominations Abilwax 9800, 9801 ou 9810 (GOLDSCHMIDT), KF910 et KF7002 (SHIN ETSU), ou 176-1118-3 et 176-11481 (GENERAL ELECTRIC).

Les cires de silicone utilisables peuvent également être choisies parmi les composés de formule (IV) :

$$R_1-Si(CH_3)_2-O-[Si(R)_2-O-]_z-Si(CH_3)_2-R_2 \qquad (IV)$$

dans laquelle :

R est défini comme précédemment,

$R_1$ représente un groupement alkyle ayant de 1 à 30 atomes de carbone, un groupement alcoxy ayant de 6 à 30 atomes de carbone, ou un groupement de formule :

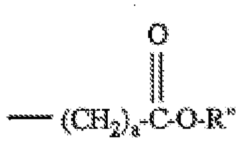

ou

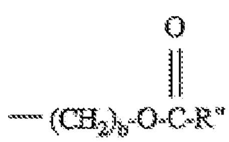

R2 représente un groupement alkyle ayant de 6 à 30 atomes de carbone, un groupement alcoxy ayant de 6 à 30 atomes de carbone ou un groupement de formule :

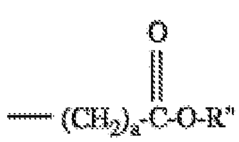

ou

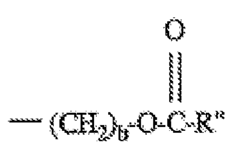

a et b représentant un nombre de 0 à 6,

R" étant un alkyle ayant de 6 à 30 atomes de carbone,

et z est un nombre pouvant varier de 1 à 100.

Parmi les cires de silicone de formule (IV), on citera notamment les alcoxydiméthicones tels que les produits commerciaux suivants : Abilwax 2428, 2434 et 2440 (GOLDSCHMIDT), ou VP 1622 et VP 1621 (WACKER), ainsi que les (C20-C60) alkyldiméthicones, en particulier les (C30-C45) alkyldiméthicones, comme la cire siliconée vendue sous la dénomination SF-1642 par la société GE-Bayer Silicones.

On peut également utiliser des cires hydrocarbonées modifiées par des groupement siliconés ou fluorés comme par exemple : siliconyl candelilla, siliconyl beeswax et Fluorobeeswax de Koster Keunen.

Les cires fluorées et/ou siliconées peuvent être présentes dans la composition en une teneur allant de 0,1 à 50 % en poids, par rapport au poids total de la composition, de préférence allant de 1 à 40 % en poids, et préférentiellement

allant de 5 à 25 % en poids.

Pigments présentant un enrobage ou un traitement de surface siliconé et/ou fluoré

Toujours dans le but d'atteindre l'énergie de surface requise, la première composition peut comprendre des pigments traités avec un traitement fluoré et/ou siliconé.

Plus particulièrement, les pigments peuvent être enrobés d'un composé organique fluoré ou d'un composé siliconé. Ils peuvent comprendre un mélange desdits pigments traités avec un traitement fluoré et/ou siliconé.

Le composé organique fluoré enrobant le pigment peut notamment être un perfluoroalkylphosphate ou un polyoxyde d'hexafluoropropylène.

Des pigments enrobés de perfluoroalkyle phosphate sont notamment décrits dans les documents US 3 632 744, JP-A- 04-330007, JP-A-2001-316223.

Le perfluoroalkyle phosphate peut notamment être choisi parmi ceux de formule (I) :

$$[C_mF_{2m+1}C_nH_{2n}O]_yPO(OM)_{3-y}$$

avec m entier allant de 1 à 21 ; n entier allant de 1 à 14 ; y = 1, 2 ou 3 ; M est -H, un cation de métal alcalin, un groupe ammonium ou un groupe ammonium substitué par un à trois radicaux alkyle en $C_1$-$C_8$.

Comme pigments enrobés de phosphate de perfluoroalkyle on peut citer la famille des Covafluor de LCW-Wackherr et le PF 5 BLACK BL 100 de Daito Kasei Kogyo.

Selon un autre mode de réalisation, le pigment peut être enrobé d'un composé siliconé, comme les méthicones, les diméthicones, les polyorganosiloxanes comprenant des groupes perfluoroalkyles perfluoropolyéthers et les perfluoroalkyl silanes.

Comme pigments enrobés, on peut par exemple utiliser dans le cadre de la présente invention :

- les pigments enrobés de phosphate de perfluoroalkyle et de copolymère acrylate de butyle/acrylate de perfluoroalkyl ($C_6$-$C_{14}$) éthyle/mercaptopropyldiméthicone vendues sous les dénominations « NOVATECH NFP Double Treated Talc », « NOVATECH NFP Double Treated Sericite », « NOVATECH NFP Double Treated Yellow Iron Oxide », « NOVATECH NFP Double Treated Red Iron Oxide », « NOVATECH NFP Double Treated Black Iron Oxide », « NOVATECH NFP Double Treated Titanium Dioxide » par la société DAIKIN ;
- les pigments enrobés de phosphate de perfluoroalkyle, de copolymère acrylate de butyle/acrylate de perfluoroalkyl ($C_6$-$C_{14}$) éthyle/mercaptopropyldiméthicone et de copolymère méthacrylate d'hydroxyéthyl/acrylate de perfluoro alkyl($C_6$-$C_{14}$) éthyle vendues sous les dénominations « NOVATECH NFP Triple Treated Talc », « NOVATECH NFP Triple Treated Sericite », « NOVATECH NFP Triple Treated Yellow Iron Oxide », « NOVATECH NFP Triple Treated Red Iron Oxide », « NOVATECH NFP Triple Treated Black Iron Oxide », « NOVATECH NFP Triple Treated Titanium Dioxide » par la société DAIKIN.

Le composé organique fluoré ou le composé siliconé enrobant le pigment peut être présent en une teneur allant de 0,01 % à 60 % en poids, par rapport au poids total du pigment enrobé, de préférence allant de 0,05 % à 40 % en poids, et préférentiellement allant de 0,1 % à 40 % en poids.

Le pigment enrobé tel que décrit précédemment peut être présent dans la première composition selon l'invention en une teneur allant de 0,01 % à 30 % en poids, par rapport au poids total de la composition, de préférence allant de 0,1 % à 20 % en poids, et préférentiellement allant de 0,5 % à 10 % en poids.

A titre de composant permettant d'atteindre l'énergie de surface requise, la première composition peut comprendre uniquement des cires fluorées et/ou siliconées ou bien uniquement des pigments traités avec un traitement fluoré et/ou siliconé ou bien encore un mélange de cires fluorées et/ou siliconées et de pigment traités avec un traitement fluoré et/ou siliconé.

Huiles

Avantageusement, la première composition est à phase continue huileuse. Les huiles ou solvants organiques envisagées dans le cadre de la présente invention sont détaillées ci-après.

Par composition à phase continue huileuse, on entend que la composition présente une conductivité, mesurée à 25 °C, inférieure à 23 μS/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

La teneur totale en huile peut aller dans la première composition peut aller de 30 à 90 % en poids, de préférence de 40 à 80 % en poids, mieux 50 à 75 % en poids par rapport au poids total de la première composition.

Selon un autre mode de réalisation particulier, la première composition comprend au moins une huile volatile, telle que définie plus loin.

Selon un mode de réalisation particulier, lorsque la première composition comprend au moins une huile non volatile, elle présente alors une teneur en huile volatile supérieure à la teneur en huile non volatile. Ainsi, la teneur en huile volatile est avantageusement comprise entre 50 et 100 % (bornes incluses), notamment entre 60 et 100 %, préférentiellement entre 80 et 100 %, en poids, par rapport au poids total d'huile dans la première composition.

Selon un autre mode de réalisation, la première composition est anhydre. Par « anhydre », on entend une composition présentant une teneur en eau et/ou en solvant hydrosoluble inférieure ou égale à 5 %, de préférence inférieure ou égale à 3 % et mieux exempte d'eau ajoutée.

Agent structurant

La première composition peut comprendre au moins un agent structurant additionnel de la phase huileuse choisi parmi les cires additionnelles différentes des cires fluorées et/ou siliconées citées plus haut, les polymères semi-cristallins, les gélifiants lipophiles et leurs mélanges. Lesdits agents structurants sont détaillés dans le paragraphe ci-après.

De préférence, l'agent structurant additionnel est présent dans la première composition dans une teneur allant de 0,1 à 20 % en poids, notamment de 0,5 à 10 % en poids, par exemple de 1 à 10 % en poids, par rapport au poids total de la première composition.

Polymère filmogène

La première composition peut en outre contenir un polymère filmogène. Un tel polymère filmogène peut être présent dans la première composition dans une teneur en matières sèches allant de 0,1 à 20 % en poids, notamment de 0,5 à 15 % en poids, de préférence de 1 à 10 % en poids, par rapport au poids total de la première composition.

D'une façon générale, la première composition peut comprendre tout composé classiquement compris dans une composition cosmétique pour le maquillage des fibres kératiniques, et notamment un mascara, pour autant que son adjonction respecte en particulier la caractéristique requise d'énergie de surface.

Selon un mode de réalisation avantageux de l'invention, la première composition ne doit pas jouer le rôle de solvant vis-à-vis de la deuxième composition. On dit alors qu'il y a incompatibilité entre la première et la deuxième composition.

La traduction de cette caractéristique a été rendue par la mise en oeuvre d'un protocole visant à mesurer la faculté de la deuxième composition, dans le cas où cette deuxième composition présente une phase continue aqueuse, à résister à la pénétration dans une couche obtenue après séchage du dépôt formé après application de la première composition sur un substrat.

Le protocole est le suivant :

On réalise des dépôts à partir de la première composition d'épaisseur 100 $\mu$m humide sur le porte échantillon plat en bakélite d'un tensiomètre à angle de contact tel que le tensiomètre à angle de contact dynamique DAT 1100, commercialisé par la Société Fibro (Suède).

Pour obtenir un tel dépôt formé par application de la première composition, on pose à plat sur le porte échantillon du tensiomètre un pochoir métallique rectangulaire de dimensions 10 X 20 mm et d'épaisseur 100 $\mu$m ; On remplit l'intérieur du cadre du pochoir avec la composition et on arase la surface à l'aide d'une lame de microscope standard (lame en verre, dimensions 26 X 76 mm), afin d'enlever l'excédent de matière.

Après séchage de ces dépôts pendant 24 heures à température ambiante (soit 23 +/- 2 °C) et humidité relative contrôlée HR 50+/-5 %, les mesures d'angles de contact sont réalisées avec le DAT 1100 à 20 +/- 1 °C et 50 % d'humidité relative avec l'eau

Une goutte d'eau, de volume compris entre 2 et 10 microlitres, est déposée automatiquement par l'appareil sur le dépôt sec formé par la première composition. Les systèmes optiques et d'analyse d'images du tensiomètre permettent de suivre l'évolution au cours du temps de l'étalement de la goutte. L'angle de contact du liquide sur le dépôt formé par la première composition correspond à l'angle compris entre la surface de contact composition/liquide et la tangente à la goutte passant par le point de rencontre de la goutte, de l'air et de la surface. Le logiciel du tensiomètre Fibro DAT 1100 permet également de suivre l'évolution du volume de la goutte.

A l'aide du tensiomètre couplé au système optique à analyse d'images on suit l'évolution de l'angle de contact $\theta$ et du volume de la goutte pendant une minute (il est généralement possible de faire une mesure toutes les 20 ms).

On estime qu'il n'y a pas de compatibilité entre le dépôt formé par la première composition, présentant une faible énergie de surface et l'eau, principal solvant de la deuxième composition, lorsque les conditions suivantes sont réunies :

$$V_{30s} \geq 0{,}90\ V_0$$

$$\theta_{30s} \geq 60\ °$$

Avec $V_{30s}$ volume de la goutte d'eau 30 secondes après dépôt et $V_0$ volume de la goutte instantanément après le dépôt de la goutte (c'est-à-dire la première acquisition de mesure dès 20 ms et au maximum jusqu'à 0,100 seconde).
$\theta_{30s}$ est l'angle de contact mesuré après 30 secondes.

**DEUXIEME COMPOSITION**

La deuxième composition présente une énergie de surface telle que la différence entre cette énergie de surface et celle de la première composition est supérieure ou égale à 5 mN/m.
Selon un mode de réalisation particulier, cette différence peut être supérieure ou égale à 7 mN/m, voire à 10 mN/m.
La deuxième composition présente en outre une viscosité comprise entre 0,001 et 2 Pa.s. De préférence, la viscosité est comprise entre 0,001 et 1 Pa.s. Selon un mode de réalisation particulièrement préféré, la viscosité est comprise entre 0,01 et 0,5 Pa.s.
Selon un mode de réalisation préféré, la deuxième composition est une composition à phase continue aqueuse.
Par composition à phase continue aqueuse, on entend que la composition présente une conductivité mesurée à 25 °C, supérieure ou égale à 23 $\mu$S/cm (microSiemens/cm), la conductivité étant mesurée comme indiquée ci-dessus.
Selon un mode de réalisation tout particulièrement préféré, la deuxième composition comprend un agent structurant et notamment des cires telles que détaillées ci-après.
Un tel agent structurant peut être présent dans la deuxième composition dans une teneur allant de 1 à 20 % en poids, notamment de 0,5 à 10 % en poids, par exemple de 1 à 10 % en poids, par rapport au poids total de la première composition.
Avantageusement, la deuxième composition comprend en outre un polymère filmogène, tel que détaillé ci-après.
Dans tous les cas, lorsqu'il est présent, la teneur en matières sèches de polymère filmogène peut aller de 0,1 à 20 % en poids, notamment de 0,5 à 15 % en poids, de préférence de 1 à 10 % en poids, par rapport au poids total de la première composition.
Selon un mode de réalisation particulier, la deuxième composition est exempte d'agent tensioactif. Elle est également avantageusement exempte de cires siliconées et/ou fluorées ainsi que de pigments traités avec un traitement fluoré et/ou siliconé.
En effet, de tels composés peuvent être de nature à aller à l'encontre de l'énergie de surface recherchée pour cette deuxième composition.
Selon un mode de réalisation préféré, la deuxième composition comprend moins de 10 %, avantageusement moins de 5 % en poids, par rapport au poids total de la composition, de cire.
Ainsi, on privilégie les compositions exemptes de cire, notamment de sorte à éviter l'utilisation d'agent tensioactif.
Selon un autre mode de réalisation privilégié, la deuxième composition comprend des charges. La présence de telles charges peut en effet constituer une aide pour la fixation de 1'« excès de matière » comme exposé précédemment.
Aussi, selon un mode particulièrement avantageux, la deuxième composition contient des charges à faible densité.
Par « charge de faible densité » on entend au sens de la présente invention des charges dont la densité tassée, mesurée par le protocole donné ci-après, varie de 0,01 à 0,80 g/cm$^3$, notamment allant de 0,05 à 0,80 g/cm$^3$, et en particulier allant de 0,10 à 0,80 g/cm$^3$.

*Protocole de mesure de la densité tassée ($D_T$) :*

Dans une éprouvette graduée de 250 ml ayant une masse $M_0$ (en g), on verse, à l'aide d'un entonnoir, un volume de poudre compris entre 240 et 250 ml. On lit ensuite sur l'éprouvette le volume $V_0$ (en cm$^3$) de poudre versée et on pèse l'éprouvette remplie de poudre pour mesurer la masse $M_1$ (en g).
Puis on place l'éprouvette sur l'appareil STAV 2003® de chez Stampf Volumeter. L'éprouvette est ensuite

soumise à une série de 2 500 tassements, puis on mesure à la fin de chaque série le volume $V_n$ (volume après la $n^{ième}$ série de 2 500 tassements) de la poudre dans l'éprouvette.

Dès que $V_n - V_{n+1} \leq (2 \times V_n) / 100$, on arrête le tassement de l'éprouvette et on note le volume $V_n$ (en $cm^3$).

La densité tassée est déterminée selon la relation suivante :

$$D_T = (M_1 - M_0) / V_n$$

Des exemples de valeurs de densité tassée sont rapportés dans le tableau qui suit :

| Charges de faible densité | Densité tassée ($g/cm^3$) |
|---|---|
| Sunsphère H-33® de la société Asahi Glass | 0,14 |
| Trefil Powder E-506C® de la société Dow Corning | 0,27 |
| KSP-100® de la société Shin Etsu | 0,39 |
| Silica Beads SB 700® de la société Maprecos | 0,48 |
| Sunsphère H-51® de la société Asahi Glass | 0,49 |
| Micropearl M-100® de la société SEPPIC | 0,64 |
| Plastic Powder D-400® de la société Toshiki | 0,76 |
| Polytrap 6603 Adsorber® de la société RP Scherrer | 0,07 à 0,10 |

Parmi les charges à faible densité, typiquement de forme sphérique, on peut notamment citer :

- les microsphères de silice, notamment à porosité ouverte, et en particulier les microsphères de silice creuses, telles que les 'SILICA BEADS SB 700/HA®' ou 'SILICA BEADS SB 700®' de la société MAPRECOS, les 'SUNSPHERES H-33®' et les 'SUNSPHERES H-51®' de la société ASAHI GLASS, les Sunsil 130 de Sunjin Chemicals ; ces microsphères peuvent, le cas échéant, être imprégnées d'un actif cosmétique
- les microsphères microporeuses de polymères, qui ont une structure analogue à celle d'une éponge ; elles ont, en général, une surface spécifique d'au moins 0,5 $m^2/g$ et en particulier d'au moins 1 $m^2/g$, ladite surface spécifique n'ayant pas de limite supérieure autre que celle résultant de la possibilité pratique de réaliser des microsphères de porosité très élevée : la surface spécifique peut, par exemple, atteindre 1 000 $m^2/g$ ou même davantage. A titre illustratif de ces microsphères, on peut citer les microsphères de polymères acryliques, telles que celles en copolymère d'acrylate réticulé 'POLYTRAP 6603 ADSORBER®' de la société RP SCHERRER, et celles de polyméthacrylate de méthyle 'MICROPEARL M 100®' de la société SEPPIC,
- la poudre de polyuréthane telle que la poudre de copolymère de diisocyanate d'hexaméthylène et de triméthylol hexyl lactone vendue sous la dénomination « PLASTIC POWDER D-400® » par la société Toshiki,
- les microcapsules de polymères qui comportent une seule cavité fermée et forment un réservoir, qui peut contenir un liquide, notamment un actif cosmétique ; elles sont préparées par des procédés connus tels que ceux décrits dans le brevet US-A 3 615 972 et EP-A 0 56219. Elles peuvent être réalisées, par exemple, en polymères ou copolymères d'acides, d'amines ou d'esters monomères à insaturation éthylénique, en polymères urée-formaldéhyde, en polymères ou copolymères de chlorure de vinylidène; à titre d'exemple, on peut citer les microcapsules faites de polymères ou copolymères d'acrylate ou de méthacrylate de méthyle, ou encore de copolymères de chlorure de vinylidène et d'acrylonitrile, comme l' « EXPANCEL® » de la société EXPANCEL ; parmi ces derniers, on indiquera, notamment, ceux qui contiennent, en poids 20-60 % de motifs dérivés de chlorure de vinylidène, 20-60 % en poids de motifs dérivés d'acrylonitrile et 0-40 % en poids d'autres motifs tels que des motifs dérivés d'un monomère acrylique et/ou styrénique; on peut également utiliser des polymères ou copolymères acryliques réticulés,
- les poudres d'organopolysiloxane réticulés élastomères, notamment décrites dans le document JP-A-02-243612, telles que celles vendues sous la dénomination «TREFIL POWDER E-506C» par la société DOW CORNING, les poudres d'organopolysiloxane réticulé élastomère enrobées de résine de silicone, notamment de résine silsesquioxane, comme décrit par exemple dans le brevet US5538793. De tels élastomère sont vendus sous les dénomination "KSP-100", "KSP-101", "KSP-102", "KSP-103", KSP-104", "KSP-105" par la société Shin Etsu, et
- leurs mélanges.

On peut également citer les particules concaves sous forme de portions de sphères creuses constituées d'un matériau organosiliconé comme les particules constituées de l'organosilicone réticulé TAK-110 (polymère réticulé méthylsilanol/silicate) commercialisées par la société TAKEMOTO OIL & FAT , en forme de bol.

La deuxième composition selon l'invention peut comprendre une charge ou un mélange de charges de faible densité en une teneur allant de 0,1 à 50 % en poids, notamment de 0,5 à 40 % en poids, et en particulier de 1 à 30 % en poids par rapport au poids total de la deuxième composition.

Outre les ingrédients précités, la deuxième composition comprise dans le kit de maquillage selon l'invention peut comprendre d'autres additifs classiques dans le domaine de la cosmétique sous réserve que leurs quantités respectives ne portent pas préjudice à la caractérisation par l'énergie de surface et la viscosité de la deuxième composition.

Ainsi, lorsqu'elle se présente sous forme d'une composition à phase continue aqueuse, la deuxième composition peut comprendre un gélifiant hydrosoluble tel que décrit plus loin.

Les polymères filmogènes hydrosolubles cités ci-après peuvent également jouer le rôle de gélifiant hydrosoluble. A ce titre, on peut par exemple citer l'hydroxyéthylcellulose et la gomme arabique.

Le polymère gélifiant hydrosoluble peut être présent dans la deuxième composition en une teneur en matière sèche allant de 0,01 % à 60 % en poids, de préférence de 0,25 % à 40 % en poids, mieux de 0,5 % à 30 % en poids, voire de 1 à 20 % en poids par rapport au poids total ou de la deuxième composition.

Selon un autre mode de réalisation, la deuxième composition est anhydre.

## HUILES

Les première et deuxième compositions comprises dans le kit de maquillage selon l'invention peuvent comprendre une ou plusieurs huiles ou solvant organique.

La ou les huiles présente(s) dans la composition de l'invention peu(ven)t être choisie(s) parmi les huiles volatiles et/ou les huiles non volatiles, et leurs mélanges.

Par « huile ou solvant organique volatile », on entend une huile ou solvant organique (ou milieu non aqueux) susceptible de s'évaporer au contact de la peau en moins d'une heure, à température ambiante et pression atmosphérique.

L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa ($10^{-3}$ à 300 mm de Hg), et de préférence allant de 1,3 Pa à 8 000 Pa (0,01 à 60 mm de Hg).

En particulier, les huiles volatiles sont choisies parmi les huiles possédant une vitesse d'évaporation supérieure ou égale à 0,002 mg/cm$^2$/min. La vitesse d'évaporation étant mesurée comme suit :

On introduit dans un cristallisoir (diamètre : 7 cm) placée sur une balance se trouvant dans une enceinte d'environ 0,3 m$^3$ régulée en température (25 °C) et en hygrométrie (humidité relative 50 %) 15 g d'huile ou du mélange d'huiles à tester.

On laisse le liquide s'évaporer librement, sans l'agiter, en assurant une ventilation par un ventilateur (vitesse de rotation 2700 tours/minute et de dimensions 80X80X42 mm, par exemple la référence 8550 N de PAPST-MOTOREN, le débit correspond à environ 50 m$^3$/heure) disposé en position verticale au-dessus du cristallisoir contenant le solvant, les pales étant dirigées vers le cristallisoir et à une distance de 20 cm par rapport au fond du cristallisoir.

On mesure à intervalles réguliers la masse d'huile restant dans le cristallisoir. Les vitesses d'évaporation sont exprimées en mg d'huile évaporée par unité de surface (cm$^2$) et par unité de temps (minute).

Les huiles (ou solvants organiques) volatiles peuvent être des huiles hydrocarbonées, des huiles siliconées, des huiles fluorées ou leurs mélanges.

On entend par « huile hydrocarbonée », une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux « d'Isopars® » ou de « Permetyls® », les esters ramifiés en $C_8$-$C_{16}$, le néopentanoate d'isohexyle, et leurs mélanges. D'autres huiles hydrocarbonées volatiles comme les distillats de pétrole, notamment ceux vendus sous la dénomination « Shell Solt® » par la société SHELL, peuvent aussi être utilisées.

Comme huiles siliconées volatiles, on peut aussi utiliser les silicones volatiles, comme par exemple les huiles de silicones linéaires ou cycliques volatiles, notamment celles ayant une viscosité ≤ 6 centistokes ($6.10^{-6}$ m$^2$/s), et ayant notamment de 3 à 6 atomes de silicium, ces silicones comportant éventuellement un ou plusieurs groupes alkyles ou alkoxy ayant de 1 ou 2 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, le 3-butyl 1,1,1,3,5,5,5-heptaméthyl trisiloxane, le 3-propyl 1,1,1,3,5,5,5-heptaméthyl trisiloxane, le 3-éthyl 1,1,1,3,5,5,5-heptaméthyl trisiloxane, et leurs mélanges.

On peut également utiliser des solvants organiques volatils notamment fluorés tels que le nonafluorométhoxy butane ou le perfluorométhylcyclopentane.

Les huiles de silicone non volatiles utilisables dans les première et deuxième compositions selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

Les huiles fluorées utilisables dans les première et deuxième compositions de l'invention sont notamment des huiles fluorosiliconées, des polyéthers fluorés, des silicones fluorées telles que décrit dans le document EP-A-847752

Avantageusement, la première composition comprend au moins une huile volatile.

Avantageusement, la ou les huile(s) volatile(s) est ou sont choisie(s) parmi les huiles volatiles hydrocarbonées ayant de 8 à 16 atomes de carbone, telle que l'isododécane, les huiles volatiles siliconées telles que le décaméthyl cyclopentasiloxane (D5), le dodécaméthyl cyclohexasiloxane (D6) et leurs mélanges.

Les première et deuxième compositions selon l'invention peuvent également comprendre au moins un composé non volatil, non soluble dans l'eau et liquide à température ambiante, notamment au moins une huile ou solvant organique non volatile, qui peut être en particulier choisie parmi les huiles hydrocarbonées et/ou siliconées et/ou fluorées non volatiles.

Comme huile hydrocarbonée non volatile, on peut notamment citer :

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces derniers pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société STEARINERIES DUBOIS ou ceux vendus sous les dénominations de Miglyol 810®, 812® et 818® par la société DYNAMIT NOBEL,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq 10$, comme par exemple l'huile de Purcellin (octanoate de cétostéaryle), le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ; et leurs mélanges.

Les huiles de silicone non volatiles utilisables dans les première et deuxième compositions selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

Les huiles fluorées utilisables dans les première et deuxième compositions de l'invention sont notamment des huiles fluorosiliconées, des polyéthers fluorés, des silicones fluorées telles que décrit dans le document EP-A-847752.

## AGENT STRUCTURANT

Les première et deuxième compositions comprises dans le kit de maquillage selon l'invention peuvent comprendre au moins un agent structurant de la phase huileuse ou solvant organique choisi parmi les cires, les polymères

semi-cristallins, les gélifiants lipophiles et leurs mélanges.
La quantité en structurant huileux peut être ajustée par l'homme du métier en fonction des propriétés de structuration desdits agents.

Cire(s)

La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.
En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.
En particulier, les cires convenant à l'invention peuvent présenter un point de fusion supérieur ou égal à 45 °C, et en particulier supérieur ou égal à 55 °C.
Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.
Le protocole de mesure est le suivant :
Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.
Les cires convenant à l'invention sont choisies parmi les cires solides à température ambiante d'origine animale, végétale, minérale ou de synthèse et leurs mélanges.
Les cires pouvant être utilisées dans les compositions cosmétiques présentent généralement une dureté allant de 0,01 MPa à 15 MPa, notamment supérieure à 0,05 MPa et en particulier supérieure à 0,1 MPa.
La dureté est déterminée par la mesure de la force en compression mesurée à 20 °C à l'aide du texturomètre vendu sous la dénomination TA-XT2 par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.
Le protocole de mesure est le suivant :
La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté ou du collant.
Le mobile du texturomètre est déplacé à la vitesse de 0,1 mm/s, puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,5 mm/s.
La valeur de la dureté est la force de compression maximale mesurée divisée par la surface du cylindre du texturomètre en contact avec la cire.
A titre illustratif des cires convenant à l'invention, on peut notamment citer les cires hydrocarbonées comme la cire d'abeilles, la cire de lanoline, et les cires d'insectes de Chine; la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters.
On peut aussi citer des cires obtenues par hydrogénation catalytique d'huiles animales ou végétales ayant des chaînes grasses, linéaires ou ramifiées, en $C_8$-$C_{32}$. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.
On peut également utiliser les cires obtenues par transesterification et hydrogénation d'huiles végétales, telles que l'huile de ricin ou d'olive, comme les cires vendues sous les dénominations de Phytowax ricin 16L64® et 22L73® et phytowax Olive 18L57 par la société SOPHIM. De telles cires sont décrites dans la demande FR-A- 2792190.
Dans la présente invention, on peut également utiliser des cires fournies sous forme de petites particules ayant

une dimension exprimée en diamètre « effectif » moyen en volume D[4,3] de l'ordre de 0,1 à 20 micromètres, en particulier de 0,2 à 10 micromètres, et plus particulièrement de 0,5 à 1 micromètres, désignées par la suite par l'expression « micro cires ». A des fins de distinction, les cires mises en oeuvre selon l'invention sous forme de fragments de dimension plus élevée sont par la suite désignées par l'expression « cires de type traditionnel ».

Les tailles de particules peuvent être mesurées par différentes techniques, on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.

De préférence, les tailles et les distributions de tailles des particules des compositions cosmétiques, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., « Light Scattering by Small Particles », Chapitres 9 et 10, Wiley, New York, 1957.

La composition cosmétiques est caractérisée par son diamètre « effectif » moyen en volume D[4,3], défini de la manière suivante :

$$D[4,3] = \frac{\sum_i V_i \cdot d_i}{\sum_i V_i}$$

où $V_i$ représente le volume des particules de diamètre effectif $d_i$. Ce paramètre est notamment décrit dans la documentation technique du granulomètre.

Les mesures sont réalisées à 25 °C, sur une dispersion de particules diluée, obtenue à partir de la composition cosmétique de la manière suivante : 1) dilution d'un facteur 100 avec de l'eau, 2) homogénéisation de la solution, 3) repos de la solution durant 18 heures, 4) récupération du surnageant homogène blanchâtre.

Le diamètre « effectif » est obtenu en prenant un indice de réfraction de 1,33 pour l'eau et un indice de réfraction moyen de 1,42 pour les particules.

Comme micro cires pouvant être utilisées dans les première et deuxième compositions conformes à l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 114S® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200®, 220®, 220L® et 250S® par la société MICRO POWDERS et les micro cires de polytétrafluoroéthylène telles que celles commercialisées sous les dénominations de Microslip 519® et 519 L® par la société MICRO POWDERS.

Dans les première et deuxième compositions comprises dans le kit de maquillage selon l'invention, on peut bien entendu utiliser un mélange de cires et notamment utiliser une ou plusieurs cires de type traditionnel et une ou plusieurs cires dites micro cires.

*Gélifiants lipophiles*

Les gélifiants utilisables dans les première et deuxième compositions comprises dans les kits de maquillage selon l'invention peuvent être des gélifiant lipophiles organiques ou minéraux, polymériques ou moléculaires.

Comme gélifiant lipophile minéral, on peut citer les argiles éventuellement modifiées comme les hectorites modifiées par un chlorure d'ammonium d'acide gras en $C_{10}$ à $C_{22}$, comme l'hectorite modifiée par du chlorure de distéaryl di-méthyl ammonium telle que, par exemple, celle commercialisée sous la dénomination de Bentone 38V® par la société ELEMENTIS.

On peut également citer la silice pyrogénée éventuellement traitée hydrophobe en surface dont la taille des particules est inférieure à 1 μm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :

- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (6ème édition, 1995). Elles sont par exemple commercialisées sous les références Aerosil R812® par la société DEGUSSA, CAB-O-SIL TS-530® par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (6ème édition, 1995). Elles sont par exemple commercialisées sous les références Aerosil R972®, et Aerosil R974® par la société DEGUSSA, CAB-O-SIL TS-610® et CAB-O-SIL TS-720® par la société CABOT.

La silice pyrogénée hydrophobe présente en particulier une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les dénominations de KSG6®, KSG16® et de KSG18® par la société SHIN-ETSU, de Trefil E-505C® et Trefil E-506C® par la société DOW-CORNING, de Gransil SR-CYC®, SR DMF10®, SR-DC556®, SR 5CYC gel®, SR DMF 10 gel® et de SR DC 556 gel® par la société GRANT INDUSTRIES, de SF 1204® et de JK 113® par la société GENERAL ELECTRIC ; l'éthylcellulose comme celle vendue sous la dénomination Ethocel® par la société DOW CHEMICAL ; les polycondensats de type polyamide résultant de la condensation entre ($\alpha$) au moins un acide choisi parmi les acides dicarboxyliques comprenant au moins 32 atomes de carbone tels que les acides gras dimères et ($\beta$) un alkylène diamine et en particulier l'éthylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG® par la société ARIZONA CHEMICAL; les galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$ et leurs mélanges. Les copolymères séquencés de type « dibloc », « tribloc » ou « radial » du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton® par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel® comme par exemple le mélange de copolymère tribloc butylène/éthylène/styrène et de copolymère étoile éthylène/propylène/styrène dans l'isododécane (Versagel M 5960).

Parmi les gélifiants lipophiles pouvant être utilisés dans les compositions cosmétiques comprises dans les kits de maquillage selon l'invention, on peut encore citer les esters de dextrine et d'acide gras, tels que les palmitates de dextrine, notamment tels que ceux commercialisés sous les dénominations Rheopearl TL® ou Rheopearl KL® par la société CHIBA FLOUR.

## POLYMERE FILMOGENE

La première et la deuxième composition comprises dans le kit de maquillage selon l'invention peuvent comprendre selon un mode de réalisation particulier au moins un polymère filmogène.

Dans la présente invention, on entend par « polymère filmogène », un polymère apte à former à lui seul ou en présence d'un agent auxiliaire de filmification, un film macroscopiquement continu et adhérent sur les fibres kératiniques, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconée.

Parmi les polymères filmogènes utilisables dans la première et la deuxième compositions comprises dans le kit selon l'invention, on peut citer les polymères synthétiques, de type radicalaire ou de type polycondensat, les polymères d'origine naturelle, et leurs mélanges.

### *Polymères liposolubles*

Selon une variante de réalisation, le polymère filmogène peut être un polymère solubilisé dans la phase huileuse comprenant des huiles ou solvants organiques tels que ceux décrits précédemment (on dit alors que le polymère filmogène est un polymère liposoluble).

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé,

linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2000 à 500 000 et de préférence de 4000 à 200 000.

Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de « MDTQ », la résine étant décrite en fonction des différentes unités monomèriques siloxane qu'elle comprend, chacune des lettres « MDTQ » caractérisant un type d'unité.

A titre d'exemples de résines polymethylsilsesquioxanes commercialement disponibles, on peut citer celles qui sont commercialisés :

- par la société Wacker sous la référence Resin MK tels que la Belsil PMS MK:
  - par la société SHIN-ETSU sous les références KR-220L.

Comme résines siloxysilicates, on peut citer les résines trimethylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination « KF-7312J » par la société Shin-Etsu, « DC 749 », « DC 593 » par la société Dow Corning.

On peut également utiliser les polyamides siliconés du type polyorganosiloxane tels que ceux décrits dans les documents US-A-5 874 069, US-A-5,919,441, US-A-6,051,216 et US-A-5,981,680.

Ces polymères siliconés peuvent appartenir aux deux familles suivantes :

1) des polyorganosiloxanes comportant au moins deux groupes capables d'établir des interactions hydrogène, ces deux groupes étant situés dans la chaîne du polymère, et/ou
2) des polyorganosiloxanes comportant au moins deux groupes capables d'établir des interactions hydrogène, ces

deux groupes étant situés sur des greffons ou ramifications.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre.

De tels polymères sont décrits par exemple dans les documents EP 1 411 069 ou WO 04/028488.

*Polymères hydrosolubles*

Selon une autre variante de réalisation, le polymère filmogène peut être un polymère hydrosoluble. Le polymère est alors solubilisé dans la phase aqueuse de la composition. Comme exemples de polymères filmogènes hydrosolubles, on peut citer :

- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :
    - les gommes arabiques, la gomme de guar, les dérivés du xanthane, la gomme de karaya ;
    - les alginates et les carraghénanes ;
    - les glycoaminoglycanes, l'acide hyaluronique et ses dérivés ;
    - la résine shellac, la gomme de sandaraque, les dammars, les élémis, les copals ;
    - l'acide désoxyribonucléique ;
    - les muccopolysaccharides tels les chondroïtines sulfate,

et leurs mélanges.

*Polymères d'origine naturelle*

Les polymères d'origine naturelle, éventuellement modifiés, peuvent être choisis parmi la résine shellac, la gomme de sandaraque, les dammars, les élémis, les copals, les polymères cellulosiques, et leurs mélanges.

*Polymères sous forme dispersée*

Le polymère filmogène peut être présent sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

a) Dispersion aqueuse

Comme dispersion aqueuse de polymère filmogène, on peut utiliser les dispersions acryliques vendues sous les dénominations Neocryl XK-90®, Neocryl A-1070®, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer Allianz Opt® par la société Rohm and Haas ou encore les dispersions aqueuses de polyuréthane vendues sous les dénominations Neorez R-981® et Neorez R-974® par la société AVECIA-NEORESINS, les Avalure UR-405®, Avalure UR-410®, Avalure UR-425®, Avalure UR-450®, Sancure 875®, Avalure UR-445® et Sancure 2060® par la société NOVEON, Impranil 85® par la société BAYER, Aquamere H-1511® par la société HYDROMER ; les sulfopolyesters vendus sous le nom de marque Eastman AQ® par la société EASTMAN CHEMICAL PRODUCTS, les dispersions vinyliques comme le Mexomère

PAM®, les dispersions aqueuses de polyvinyl acétate comme le « Vinybran® » de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylméthacrylamidoammonium telles que le Styleze W-d'ISP, les dispersions aqueuses de polymères hybrides polyuréthane/polyacryliques telles que celles commercialisées sous les références « Hybridur® » par la société AIR PRODUCTS ou « Duromer® » de NATIONAL STARCH, les dispersions type core/shell : par exemple celles commercialisées par la société ATOFINA sous la référence Kynar (core : fluoré-shell : acrylique) ou encore ceux décrits dans le document US 5 188 899 (core ; silice - shell : silicone) et leurs mélanges.

b) Dispersion non aqueuse

On peut aussi citer les dispersions de particules d'un polymère éthylénique greffé, de préférence acrylique, dans une phase huileuse liquide, le polymère éthylénqiue étant avantageusement dispersé en l'absence de stabilisant additionnel en surface des particules telles que décrite notamment dans le document WO 04/055081.
Les première et deuxième compositions conformes à l'invention peuvent comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.
A ce titre, on peut citer les dispersions acryliques dans l'isododécane comme le Mexomère PAP® par la société CHIMEX.

## EAU ET/OU SOLVANT HYDROSOLUBLE

Par "solvant hydrosoluble", on désigne dans la présente invention un composé liquide à température ambiante et miscible à l'eau (miscibilité dans l'eau supérieure à 50 % en poids à 25 °C et pression atmosphérique).
Parmi les solvants hydrosolubles pouvant être utilisés dans lesdites compositions cosmétiques, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en $C_3$ et $C_4$ et les aldéhydes en $C_2$-$C_4$.
La phase aqueuse (eau et/ou solvant(s) hydrosoluble(s)) peut être introduite en tant que telle dans les première et deuxième compositions comprises dans le kit de maquillage ou y être incorporée par le biais, d'un ou plusieurs ingrédients constituant lesdites première et deuxième compositions. Ainsi de l'eau peut être notamment introduite dans une des compositions par le biais de l'introduction de latex ou de pseudolatex, c'est-à-dire de dispersion aqueuse de particules de polymère.

## MATIERES COLORANTES

Les première et deuxième compositions comprises dans les kits de maquillage selon l'invention peuvent indépendamment l'une de l'autre également comprendre au moins une matière colorante comme les matières pulvérulentes, les colorants liposolubles, les colorants hydrosolubles.
Les matières colorantes pulvérulentes peuvent être choisies parmi les pigments et les nacres.
Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.
Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.
Ces matières colorantes peuvent être présentes en une teneur allant de 0,01 à 30 % en poids par rapport au poids total de chacune desdites première et deuxième compositions.
Les matières colorantes peuvent être similaires ou différentes dans la première et deuxième composition. Il est aussi possible, en jouant sur les teintes des compositions de personnaliser les rendus maquillages à volonté.
Ainsi selon un mode de réalisation, les compositions peuvent présenter des teintes différentes. Par exemple l'une des compositions peut être transparente ou translucide et l'autre composition, colorée. Selon une variante, les première et deuxième compositions peuvent présenter des couleurs différentes.

Selon une variante, la deuxième composition peut avantageusement comprendre des nacres ou des paillettes, telles que des particules réfléchissantes.
Les deux compositions peuvent également différer par toute caractéristique optique visible à l'oeil nu, telle que la brillance.

## ADDITIFS

### Fibres

Les première et deuxième compositions comprises dans les kits de maquillage selon l'invention peuvent indépendamment l'une de l'autre également en outre comprendre des fibres qui permettent notamment une amélioration de l'effet allongeant dans le domaine du maquillage des fibres kératiniques.
Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.
Les fibres utilisables dans les compositions comprises dans les kits de maquillage de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.
En particulier, les fibres ont une longueur allant de 1 $\mu$m à 10 mm, en particulier de 0,1 mm à 5 mm et plus particulièrement de 0,3 mm à 3,5 mm. Leur section peut être comprise dans un cercle de diamètre allant de 2 nm à 500 $\mu$m, en particulier allant de 100 nm à 100 $\mu$m et plus particulièrement de 1 $\mu$m à 50 $\mu$m. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.
Les fibres utilisables dans les compositions comprises dans les kits de maquillage selon l'invention peuvent être choisies parmi les fibres rigides ou non rigides, elles peuvent être d'origine synthétique ou naturelle, minérales ou organiques.
Par ailleurs, les fibres peuvent être traitées ou non en surface, enrobées ou non, colorées ou non colorées.
A titre de fibres utilisables dans les compositions comprises dans les kits de maquillage selon l'invention, on peut citer les fibres non rigides telles que les fibres de polyamide (Nylon®) ou les fibres rigides telles que les fibres de polyimide-amide comme celles vendues sous les dénomination KERMEL®, KERMEL TECH® par la société RHODIA ou de poly-(p-phénylène-téréphtalamide) (ou d'aramide) notamment vendues sous la dénomination Kevlar® par la société DUPONT DE NEMOURS.
Des fibres peuvent êtres présentes dans la première ou la deuxième composition en une teneur allant de 0,01 % à 10 % en poids, par rapport au poids total de la composition les comprenant, en particulier de 0,05 % à 5 % en poids, et plus particulièrement de 0,1 % à 3 % en poids.
En réalité, lorsqu'elles sont présentes, les fibres le sont préférentiellement dans la première composition.

### Actifs cosmétiques

Les première et deuxième compositions comprises dans les kits de maquillage selon l'invention peuvent indépendamment l'une de l'autre également, de plus, comprendre tous les ingrédients classiquement utilisés en cosmétique. Ces ingrédients peuvent notamment être choisis parmi les polymères, notamment les polymères fixants ; les opacifiants ; les parfums ; les épaississants ; les gélifiants ; les colorants capillaires ; les résines de silicone ; les gomme de silicone ; les conservateurs ; les antioxydants, les actifs cosmétiques ; les filtres solaires ; les agents de stabilisation du pH ; les vitamines ; les hydratantset leurs mélanges. Les quantités de ces différents ingrédients sont celles classiquement utilisées dans les domaines concernés et par exemple de 0,01 à 20 % en poids par rapport au poids total de la première composition et de 0,1 à 10 % en poids par rapport au poids total de la deuxième composition.
Bien entendu, l'homme du métier veillera à choisir ce ou ces éventuels additifs, et/ou leur quantité, de manière telle que les propriétés avantageuses des première et deuxième compositions selon l'invention ne soient pas, ou substantiellement pas, altérées par l'adjonction envisagée.

## APPLICATEUR ET KIT

Tout type d'applicateur peut être utilisé en vue du dépôt de la première et/ou de la seconde composition.
Les applicateurs respectifs de la première et de la seconde compositions peuvent être identiques ou distincts.
Selon une variante de l'invention, les première et seconde compositions peuvent être appliquées au moyen

de deux portions applicatrices distinctes du même applicateur.

Selon une autre variante, le kit de maquillage selon la présente invention comprend un ou plusieurs moyens d'application des première et deuxième compositions.

Les première et deuxième compositions cosmétiques comprises dans le kit de maquillage de l'invention peuvent être en particulier appliquées sur les cils, à l'aide d'une brosse, d'un peigne, d'une tige filetée ou d'un stylo muni d'une molette crantée.

En l'occurrence, la première composition peut être appliquée à l'aide de tout applicateur pour autant qu'il permet un dépôt localisé sur une partie de la longueur de la fibre kératinique et lorsqu'il s'agit d'un cil sur au plus 2/3 de sa longueur.

Il est particulièrement avantageux de procéder à l'application de la première composition avec un peigne.

Il est particulièrement avantageux de procéder à l'application de la deuxième composition à l'aide d'une tige filetée ou d'un stylo muni d'une molette crantée.

L'application de la deuxième composition à l'aide d'une tige filetée permet notamment de former des fines gouttelettes alignées plus ou moins régulièrement le long de l'extrémité des cils lorsque la première composition a été appliquée sur la base des cils.

La tige filetée peut être en matière plastique ou en métal ou tout autre matériau adapté.

Le stylo muni d'une molette crantée est plus particulièrement décrit dans la demande US 5851019 ou FR2588733.

Ce stylo permet un dosage précis de produit à appliquer à la sortie du stylo après actionnement d'une molette crantée ou d'un bouton poussoir qui fait avancer un piston à chaque rotation/pression.

Ce moyen d'application permet notamment la formation de gouttes, par exemple des gouttes uniques à l'extrémité des cils lorsque la première composition a été appliquée sur la base des cils. Les gouttes sont généralement de taille plus importante que celles obtenues par application à l'aide de la tige filetée.

Le kit de maquillage selon l'invention peut, selon un mode de réalisation particulier, comprendre au moins deux conditionnements distincts, l'un comprenant la première composition et l'autre comprenant la deuxième composition.

En réalité, le maquillage des fibres kératiniques étant effectué grâce à un geste multiple de l'utilisatrice, à savoir au moins en deux étapes, la première consistant en l'application de la composition « basecoat » et la deuxième consistant en l'application de la composition « topcoat » en tout ou partie sur la première composition dite « basecoat » et sur les fibres kératiniques, un kit de maquillage conditionné dans un seul et même conditionnement est particulièrement adapté. Cette alternative constitue un mode de réalisation préféré de l'invention. Selon un mode de réalisation particulièrement avantageux, cet article de conditionnement est muni de deux applicateurs distincts.

Lorsque le kit est sous forme d'un seul et même conditionnement, il peut se présenter comme un récipient délimitant au moins un compartiment ou réservoir qui comprend la composition « basecoat », ledit compartiment étant fermé par un élément de fermeture et au moins un compartiment ou réservoir qui comprend la composition « topcoat », étant également fermé par un élément de fermeture.

L'applicateur peut être solidaire du récipient. Avantageusement, l'applicateur est solidaire d'une tige qui, elle même, est solidaire de l'élément de fermeture.

L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par « encliquetage » on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient, comportant avantageusement deux compartiments ou réservoirs, peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient est de préférence équipé d'un essoreur disposé au voisinage d'au moins une ouverture du récipient. Un tel essoreur permet d'essuyer l'applicateur et éventuellement, la tige dont il peut être solidaire. Un tel essoreur est décrit par exemple dans le brevet FR 2 792 618.

Selon un mode de réalisation particulièrement préféré, le kit de maquillage comprend deux réservoirs comprenant chacun l'une des compositions « basecoat » et « topcoat », chacun des réservoirs étant muni d'une brosse et/ou d'une peigne et/ou d'une tige filetée et/ou d'un stylo muni d'une molette crantée, notamment le réservoir de la composition « basecoat » étant munie d'un peigne à mascara et le réservoir de la composition « topcoat » étant muni d'une tige filetée ou d'un stylo muni d'une molette crantée.

**PROCEDE DE MAQUILLAGE ET/OU DE SOIN**

La première composition peut être appliquée sur tout ou partie des fibres kératiniques, par exemple sur la moitié ou le tiers de la longueur des fibres, et en tous les cas lorsqu'il s'agit de cils au plus sur les deux tiers de leur longueur.
Ainsi, lorsqu'elle est appliquée sur la base des cils, le résultat maquillage est proche de celui représenté en figures 2 et 3.
Lorsqu'elle est appliquée sur l'extrémité des cils, le résultat maquillage est proche de celui représenté en figure 1.
La deuxième composition peut également être appliquée sur tout ou partie des fibres kératiniques, par exemple sur la moitié ou le tiers de la longueur des fibres. Elle peut être appliquée avant le séchage complet du dépôt formé par la première composition.
Par exemple, la deuxième composition peut être déposée sur la totalité de la fibre kératinique et on constate de façon surprenante que le dépôt de cette deuxième composition démouille de la surface sur laquelle a été réalisé le dépôt de la première composition, pour aller préférentiellement maquiller les zones de fibres kératiniques non couvertes par le premier dépôt.
La présente invention est donc également relative à un procédé de maquillage et/ou de soin non thérapeutique de fibres kératiniques, caractérisé en ce qu'il comprend au moins une étape d'application d'une première composition conforme à la présente invention sur tout ou partie des fibres kératiniques, formant un premier dépôt, et au moins une étape d'application d'une deuxième composition conforme à la présente invention sur tout ou partie du premier dépôt et/ou des fibres kératiniques.
La présente invention vise également un procédé de maquillage et/ou de soin non thérapeutique des fibres kératiniques destiné à former des perles ou des ponts à l'extrémité desdits fibres kératiniques sur lesdites fibres kératiniques, caractérisé en ce qu'il comprend au moins une étape d'application sur la base des fibres kératiniques d'une première composition conforme à la présente invention formant un premier dépôt, et au moins une étape d'application d'une deuxième composition conforme à la présente invention sur tout ou partie du premier dépôt et/ou des fibres kératiniques.
La présente invention vise enfin un procédé de maquillage et/ou de soin non thérapeutique des fibres kératiniques, destiné à former des ponts à proximité de la base desdites fibres kératiniques, caractérisé en ce qu'il comprend au moins une étape d'application sur l'extrémité des fibres kératiniques d'une première composition conforme à la présente invention formant un premier dépôt et au moins une étape d'application d'une deuxième composition sur tout ou partie du premier dépôt et/ou des fibres kératiniques.
L'invention pourra être mieux comprise à l'examen du dessin annexé sur lequel :

- la figure 1 représente une portion de franges de cils maquillés selon une des alternatives du procédé de maquillage conforme à la présente invention,
- la figure 2 représente une portion de franges de cils maquillés selon une autre alternative du procédé de maquillage conforme à la présente invention,
- la figure 3 représente une portion de franges de cils maquillés selon encore une autre alternative du procédé de maquillage conforme à la présente invention.

Sur l'ensemble des figures, une portion de paupière 1 est représentée.
Sur la figure 1, le maquillage a été obtenu par application de la première composition sur l'extrémité des cils sur une portion P1 qui, sur la figure 1, correspond environ au tiers de la longueur dudit cil. Cette longueur pourrait être amenée aux deux tiers de la longueur dudit cil sans changer le type de maquillage. La seconde composition a été appliquée sur la totalité de la longueur des cils. Un pont de matière 4 a été créé entre les cils au niveau de leur base.
Sur les figures 2 et 3, le maquillage a été obtenu par application de la première composition sur une portion P2, respectivement P3, des cils qui sur la figure 2, respectivement 3, correspond environ au tiers inférieur de la longueur dudit cil.
Cette portion P2, respectivement P3, pourrait être amenée aux deux tiers de la longueur dudit cil sans changer le type de maquillage. La seconde composition a été appliquée sur la totalité de la longueur des cils. Un pont de matière 4, respectivement des perles 5, ont été créés entre les cils au niveau de leur extrémité.
Dans le cas du maquillage des cils, un tel procédé peut être opéré sur tout ou partie d'une frange de cils.
Les exemples de formulation qui suivent sont précisés à titre illustratif et non limitatif de l'invention. Sauf indication contraire, les quantités sont données en pourcentage massique.

**EXEMPLES**

On peut réaliser les compositions suivantes :

Composition n°1 : formule de mascara waterproof contenant un alkyl silicone et des pigments enrobés fluorés :

- 17 % $C_{30}$-$_{45}$ alkyl diméthicone SF-1642 (GE-Bayer Silicones)
- 7 % copolymère allyl stearate/vinyl acetate
- 5 % oxyde de fer noir enrobé de phosphate de perfluoroalkyle, vendu sous la dénomination « Black Iron Oxide Covafluor » par la société Wackherr
- 5 % Quaternium 18-Bentonite (Bentone 38V® d'ELEMENTIS)
- 1,6 % propylene carbonate
- 64,4 % isododécane

Composition n°2 : formule de mascara contenant une silice creuse hydrophile :

- 1,4 % hydroxyéthyl cellulose (Cellosize QP 4400H de Union Carbide)
- 25 % silice hydrophile creuse (Sunsil 130 de Sunjin Chemicals)
- 6,69 % gomme arabique
- 3,35 % pigments (oxyde de fer noir)
- 3,35 % propylène glycol
- 0,30 % sodium méthyl paraben (conservateur)
- eau qsp 100 g

L'énergie de surface de la composition n°1 a été mesurée selon le protocole décrit ci-dessus pour la "première composition". La valeur mesurée est de 13,73 mN/m.
L'énergie de surface de la composition n°2 a été mesurée selon le protocole décrit ci-dessus pour la "deuxième composition". La valeur mesurée, sur une moyenne de trois mesures, est de 52,83 mN/m.
Composition n°3 : formule de mascara waterproof contenant une cire téflonée et des pigments enrobés PDMS.

- 20 % poudre micronisée de poly tetrafluoroethylene, vendu sous la dénomination MICROSLIP 519 L par la société Micro Powders.
- 7 % copolymère allyl stearate/vinyl acetate
- 5 % oxyde de fer brun enrobé de silice et de polydimethylsiloxane, vendu sous la dénomination SA - LUCE RED par la société Lyoshi Kasei
- 8 % Quaternium 18-Bentonite (Bentone 38VÒ d'ELEMENTIS)
- 2,6 % propylene carbonate
- 57,4 % isododécane

Composition n°4 : formule de mascara contenant des microsphères creuses de PMMA et un latex :

- 1,4 % hydroxyéthyl cellulose (Cellosize QP 4400H de Union Carbide)
- 15 % micro-sphères creuses de poly méthacrylate de méthyle (COVABEAD LH 85 de LCW)
- 6,69 % gomme arabique
- 10 % Copolymère acrylate d'éthyle/méthacrylate de méthyle réticulé, en dispersion aqueuse à 50 %, vendu sous la dénomination DAITOSOL 5000 AD par la société DAITO KASEI KOGYO
- 3,35 % pigments (oxyde de fer brun)
- 3,35 % propylène glycol
- 0,30 % sodium méthyl paraben (conservateur)
- eau qsp 100 g.

Différents maquillages sont testés sur des éprouvettes de faux cils à l'aide de deux compositions choisies parmi les compositions n° 1 à n° 4 ci-dessus.

Lorsque la composition n° 1 ou n°3 est appliquée sur la base des cils et la composition n°2 ou n°4 sur l'ensemble de la frange des cils, il y a démouillage de la composition n°2 ou n°4, qui migre vers l'extrémité des cils (zone d'énergie de surface plus élevée), de sorte à se rapprocher du résultat maquillage de la figure 3.

Par ailleurs, la composition n°1 peut être appliquée en base-coat et la composition n°4 en top-coat.

Lorsque la composition n°1 ou n°3 est appliquée sur l'extrémité des cils, il y démouillage de la composition n°2 ou n°4 qui migre vers les zones non maquillées par la composition n°1 ou n°3 (milieu et base des cils) de sorte à se rapprocher du résultat maquillage de la figure 1.

La composition n°1 peut être appliquée en base-coat et la composition n°2 en top-coat.

Alternativement, la composition n°3 peut être appliquée en base-coat et la composition n°2 en top-coat.

Encore alternativement, la composition n°3 peut être appliquée en base-coat et la composition n°4 en top-coat.

## Revendications

1. **1.** Kit de maquillage des fibres kératiniques comprenant au moins :

   - une première composition présentant une énergie de surface inférieure ou égale à 30 mN/m à 23 +/- 2°C, et
   - une deuxième composition présentant une énergie de surface, telle que la différence entre cette énergie de surface et celle de la première composition est supérieure ou égale à 5 mN/m, et une viscosité comprise entre 0,001 et 2 Pa.s à 25°C +/- 0,5 °C.

2. **2.** Kit selon la revendication 1, dans lequel la première composition présente une énergie de surface inférieure ou égale à 25 mN/m, de préférence inférieure ou égale à 20 mN/m.

3. **3.** Kit selon l'une quelconque des revendications précédentes, dans lequel la première composition comprend des composés siliconés et/ou fluorés, notamment choisis parmi les cires fluorées et/ou siliconées, les pigments présentant un enrobage ou un traitement de surface siliconé et/ou fluoré et leurs mélanges.

4. **4.** Kit selon la revendication précédente, dans lequel les cires fluorées et/ou siliconées sont choisies parmi les esters fluorés, les microdispersions de cires fluorées, les polysiloxanes linéaires substitués, les alcoxydiméthicones, les ($C_{20}$-$C_{60}$) alkyldiméthicone, et les cires hydrocarbonées modifiées par des groupements siliconés ou fluorés.

5. **5.** Kit selon la revendication précédente, dans lequel les cires fluorées et/ou siliconées sont présentes dans la première composition dans une teneur allant de 0,1 à 50 % en poids, par rapport au poids total de la composition, de préférence allant de 1 à 40 % en poids, et préférentiellement allant de 5 à 25 % en poids.

6. **6.** Kit selon l'une quelconque des revendications précédentes, dans lequel la première composition comprend des pigments traités avec un traitement fluoré et/ou siliconé.

7. **7.** Kit selon la revendication précédente, dans lequel les pigments traités avec un traitement fluoré et/ou siliconé sont choisis parmi les pigments enrobés par un perfluoroalkylphosphate, un polyoxyde d'hexafluoropropylène, et les pigments enrobés par un composé siliconé, comme les méthicones, les diméthicones, les polyorganosiloxanes comprenant des groupes perfluoroalkyles perfluoropolyéthers et les perfluoroalkyl silanes.

8. **8.** Kit selon la revendication précédente, dans lequel les pigments traités avec un traitement fluoré et/ou siliconé sont présents en une teneur allant de 0,01 % à 30 % en poids, par rapport au poids total de la composition, de préférence allant de 0,1 % à 20 % en poids, et préférentiellement allant de 0,5 % à 10 % en poids.

9. **9.** Kit selon l'une quelconque des revendications précédentes, dans lequel la première composition est à phase continue huileuse.

10. **10.** Kit selon l'une quelconque des revendications précédentes, dans lequel la première composition comprend au moins une huile non volatile.

11. **11.** Kit selon l'une quelconque des revendications précédentes, dans lequel la première composition comprend au moins une huile volatile.

12. **12.** Kit selon l'une quelconque des revendications précédentes, dans lequel la première composition présente une teneur en huile volatile supérieure à la teneur en huile non volatile, et notamment la teneur en huile volatile est comprise entre 50 et 100 %, notamment entre 60 et 100 %, préférentiellement entre 80 et 100 %, en poids, par rapport au poids total d'huile dans la première composition.

13. **13.** Kit selon l'une quelconque des revendications précédentes, dans lequel la teneur totale en huile peut aller dans la première composition de 30 à 90 % en poids, de préférence de 40 à 80 % en poids, mieux de 50 à 75 % en poids, par rapport au poids total de la première composition.

14. **14.** Kit selon l'une quelconque des revendications précédentes, dans lequel la première composition est anhydre.

**15.** Kit selon l'une quelconque des revendications précédentes, dans lequel la première composition comprend en outre au moins un agent structurant choisi parmi les cires additionnelles différentes des cires fluorées et/ou siliconées telles que définies dans la revendication 4, les polymères semi-cristallins, les gélifiants lipophiles et leurs mélanges.

**16.** Kit selon l'une quelconque des revendications précédentes, dans lequel la première composition comprend en outre au moins un polymère filmogène.

**17.** Kit selon l'une quelconque des revendications précédentes, dans lequel la deuxième composition présente une énergie de surface telle que la différence entre cette énergie de surface et celle de la première composition soit supérieure ou égale 7mN/m, voire à 10 mN/m.

**18.** Kit selon l'une quelconque des revendications précédentes, dans lequel la viscosité de la deuxième composition est comprise entre 0,001 et 1 Pa.s, de préférence entre 0,01 et 0,5 Pa.s.

**19.** Kit selon l'une quelconque des revendications précédentes, dans lequel la deuxième composition est à phase continue aqueuse.

**20.** Kit selon l'une quelconque des revendications précédentes, dans lequel la deuxième composition est exempte de cire.

**21.** Kit selon l'une quelconque des revendications précédentes, dans lequel la deuxième composition est exempte d'agent tensioactif.

**22.** Kit selon l'une quelconque des revendications précédentes, dans lequel la deuxième composition comprend au moins un polymère filmogène.

**23.** Kit selon l'une quelconque des revendications précédentes, dans lequel la deuxième composition comprend en outre des charges.

**24.** Kit selon la revendication précédente, dans lequel les charges sont des charges de faible densité.

**25.** Kit selon l'une quelconque des revendications précédentes, dans lequel la deuxième composition comprend des charges de faible densité dans une teneur allant de 0,1 à 50 % en poids, notamment de 0,5 à 40 % en poids, et en particulier de 1 à 30 % en poids, par rapport au poids total de la deuxième composition.

**26.** Kit de maquillage des fibres kératiniques selon l'une quelconque des revendications précédentes, dans lequel les première et deuxième compositions sont comprises dans un même article de conditionnement muni de deux applicateurs distincts.

**27.** Procédé de maquillage des fibres kératiniques, **caractérisé en ce qu'**il comprend au moins une étape d'application d'une première composition telle que définie selon l'une quelconque des revendications 2 à 16 sur tout ou partie des fibres kératiniques, formant un premier dépôt, et au moins une étape d'application d'une deuxième composition telle que définie selon l'une quelconque des revendications 17 à 25 sur tout ou partie du premier dépôt et/ou des fibres kératiniques.

**28.** Procédé de maquillage des fibres kératiniques destiné à former des perles ou des ponts à l'extrémité desdits fibres kératiniques sur lesdites fibres kératiniques, **caractérisé en ce qu'**il comprend au moins une étape d'application sur la base des fibres kératiniques d'une première composition telle que définie selon l'une quelconque des revendications 2 à 16, formant un premier dépôt, et au moins une étape d'application d'une deuxième composition telle que définie selon l'une quelconque des revendications 17 à 25 sur tout ou partie du premier dépôt et/ou des fibres kératiniques.

**29.** Procédé de maquillage des fibres kératiniques, destiné à former des ponts à proximité de la base desdites fibres kératiniques, **caractérisé en ce qu'**il comprend au moins une étape d'application sur l'extrémité des fibres kératiniques d'une première composition telle que définie selon l'une quelconque des revendications 2 à 16 formant un premier dépôt, et au moins une étape d'application d'une deuxième composition telle que définie selon l'une quelconque des revendications 17 à 25 sur tout ou partie du premier dépôt et/ou des fibres kératiniques.

Fig.1

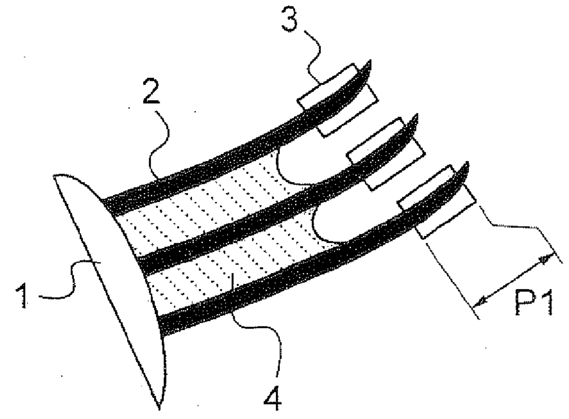


Fig.2

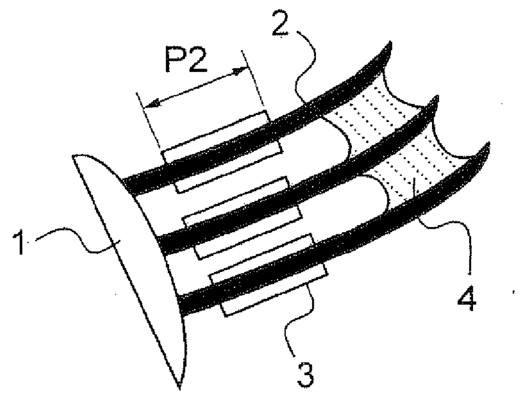


Fig.3

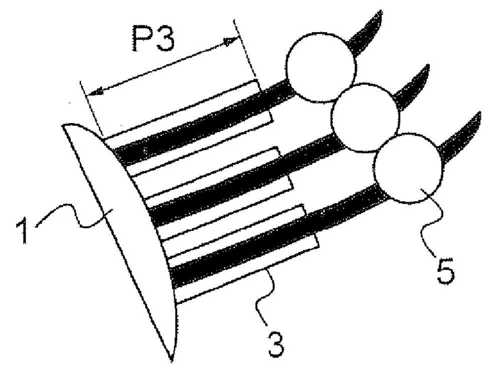

Office européen des brevets

# RAPPORT DE RECHERCHE EUROPEENNE

Numéro de la demande
EP 08 15 0029

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | EP 1 704 896 A (L'ORÉAL) 27 septembre 2006 (2006-09-27) * alinéas [0001], [0003], [0029], [0033]; revendications 1,15,20 * * alinéas [0050] - [0054], [0100], [0101], [0117], [0134], [0155], [0197], [0198] * * alinéas [0202], [0205], [0219], [0220]; exemple 1 * | 1-5,9-29 | INV. A61K8/81 A61K8/73 A61K8/891 A61K8/19 A61Q1/10 |
| Y | | 6-8 | |
| | ----- | | |
| Y | EP 1 433 462 A (L'ORÉAL) 30 juin 2004 (2004-06-30) * alinéas [0102] - [0105] * | 6-8 | |
| | ----- | | |

DOMAINES TECHNIQUES RECHERCHES (IPC)

A61K
A61Q

Le présent rapport a été établi pour toutes les revendications

| Lieu de la recherche | Date d'achèvement de la recherche | Examinateur |
|---|---|---|
| Berlin | 11 avril 2008 | Alvarez Alvarez, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..........................................................................
& : membre de la même famille, document correspondant

4
EPO FORM 1503 03.82 (P04C02)

**ANNEXE AU RAPPORT DE RECHERCHE EUROPEENNE RELATIF A LA DEMANDE DE BREVET EUROPEEN NO.** EP 08 15 0029

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-04-2008

| Document brevet cité au rapport de recherche | Date de publication | Membre(s) de la famille de brevet(s) | Date de publication |
|---|---|---|---|
| EP 1704896 A | 27-09-2006 | FR 2883470 A1 | 29-09-2006 |
| | | JP 2006265252 A | 05-10-2006 |
| EP 1433462 A | 30-06-2004 | BR 0306088 A | 17-05-2005 |
| | | FR 2848821 A1 | 25-06-2004 |
| | | MX PA04000143 A | 17-06-2005 |
| | | ZA 200309963 A | 16-08-2004 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 953332 A
- FR 2844706
- US 3632744 A
- JP 4330007 A
- JP 2001316223 A
- US 3615972 A
- EP 056219 A
- JP 2243612 A
- US 5538793 A
- EP 847752 A
- FR 2792190 A
- FR 2232303 A
- US 5874069 A
- US 5919441 A
- US 6051216 A
- US 5981680 A
- EP 1411069 A
- WO 04028488 A
- US 5188899 A
- WO 04055081 A
- US 5851019 A
- FR 2588733
- FR 2792618


### Littérature non-brevet citée dans la description


- **FOWKES, F. M.** Determination of interfacial tensions, contact angles, and dispersion forces in surfaces by assuming additivity of intermolecular interactions in surfaces. *Journal of Physical Chemistry,* 1962, vol. 66, 382
- Contact angle, wettability & adhesion, advances in chemistry series. *Fowkes,* 1964, (43
- **VAN DE HULST, H.C.** Light Scattering by Small Particles. Wiley, 1957